# EUROPEAN PATENT SPECIFICATION

(11) **EP 1 148 139 B1**
(45) Date of publication and mention of the grant of the patent: **25.08.2004**
(21) Application number: 99959952.5
(22) Date of filing: 21.12.1999
(51) Int. Cl.: C12N 15/53, C12N 9/02, C12N 1/21

(54) **VARIANT LUCIFERASE, VARIANT LUCIFERASE GENE, NOVEL RECOMBINANT DNA AND PROCESS FOR PRODUCING VARIANT LUCIFERASE**
LUZIFERASE-VARIANTE, GEN DER LUZIFERASE-VARIANTE, REKOMBINANTE DNA UND VERFAHRENZUR HERSTELLUNG DER LUZIFERASE-VARIANTE
VARIANT DE LUCIFERASE, GENE DE VARIANT DE LUCIFERASE, ADN RECOMBINANT ET TECHNIQUE PERMETTANT DE PRODUIRE UN VARIANT DE LUCIFERASE

(30) Priority: 05.01.1999 JP 26999
(43) Date of publication of application: 24.10.2001
(73) Proprietor: KIKKOMAN CORPORATION, Noda-shi, Chiba 278-8601 (JP)
(72) Inventor: KAJIYAMA, Naoki, Noda-shi, Chiba 278-8601 (JP); NAKANO, Eiichi, Noda-shi, Chiba 278-8601 (JP)
(74) Representative: Brasnett, Adrian Hugh
(86) International application number: PCT/JP1999/007192
(87) International publication number: WO 2000/040732

(56) References cited:
- EP-A- 0 449 621
- WO-A1-95/18853
- DEMENTIEVA E I ET AL: "PHYSICOCHEMICAL PROPERTIES OF RECOMBINANT LUCIOLA MINGRELICA LUCIFERASE AND ITS MUTANT FORMS" BIOCHEMISTRY, AMERICAN CHEMICAL SOCIETY. EASTON, PA, US, vol. 1, no. 61, 1996, pages 115-119, XP002078631 ISSN: 0006-2960
- DEVINE JERRY H ET AL: "Luciferase from the East European firefly Luciola mingrelica: Cloning and nucleotide sequence of the cDNA, overexpression in Escherichia coli and purification of the enzyme." BIOCHIMICA ET BIOPHYSICA ACTA, vol. 1173, no. 2, 28 May 1993 (1993-05-28), pages 121-132, XP001070250 ISSN: 0006-3002
- THOMPSON, J. F. ET. AL.: 'Mutation of a protease-sensitive region in firefly luciferase alters light emission properties.' THE JOURNAL OF BIOLOGICAL CHEMISTRY vol. 272, no. 30, 1997, pages 18766 - 18771, XP002924331
- EIICHI NAKANO: 'Mass production of firefly luciferase by Escherichia coli (in Japanese).' SCIENCE AND INDUSTRY (KAGAKU TO KOGYO) vol. 66, no. 6, 1992, pages 232 - 237, XP002929905
- MASUDA, T. ET. AL.: 'Cloning and sequence analysis of cDNA for luciferase of a Japanese firefly, Luciola cruciata.' GENE vol. 77, no. 2, 1989, pages 265 - 270, XP002924332
- SALA-NEWBY G.B. ET AL: 'Sequence and biochemical similarities between the luciferases of the glow-worm Lampyris noctiluca and the firefly Photinus pyralis' BIOCHEMISTRY JOURNAL vol. 313, 1996, pages 761 - 767

## Description

### BACKGROUND OF THE INVENTION

### Field of the Invention

The present invention relates to a variant luciferase, a variant luciferase gene, a novel recombinant DNA and a method for producing the variant luciferase.

### Background Art

Luciferase is an enzyme for light emission, which acts to catalyze the oxidation of luciferins, resulting in light emission of these luciferins (Japanese Patent Application Laid-Open (kokai) No. 1-141592 (published on June 2, 1989). Luciferases derived from Coleoptera such as a firefly, *Pyrophorus noctilucus* and *Phrixpthrix* sp. are used for the microdetermination of ATP, because Coleoptera needs substances such as the above ATP for the light emission of luciferins. Recently, it has become possible to produce a large amount of luciferase by the development of a variant firefly luciferase emitting a different-colored light from the wild type one by the genetic recombination (Japanese Patent Application Laid-Open (kokai) No. 3-285683 (published on December 16, 1991), and so the use of luciferase as a luminous plaything (International Publication WO98/28569) is also known.

However, in general, the light emission level by Coleoptera luciferase sharply decreases, after the light emission amount reachs its peak immediately after reaction, and accordingly, it was difficult to perform high sensitivity determination of ATP or the like with a prolonged reaction. Furthermore, in a case where luciferase is used as a luminous plaything, the improvement of luminescence sustention has been desirable.

Therefore, the objective of the present invention is to provide a variant Coleoptera luciferase acting to bring about stably sustained luminescence over a long period of time.

### SUMMARY OF THE INVENTION

Through intensive studies directed toward the above objective, the present inventors have found a variant Coleoptera luciferase, wherein a specific amino acid residue is substituted by another amino acid residue in a Coleoptera luciferase, which achieves the above objective, thereby completing the present invention.

That is to say, in a first aspect, the present invention provides a variant luciferase having improved luminescence sustention when compared with the wild type.

In one embodiment, the present invention provides a variant luciferase, wherein, in the amino acid sequence of luciferase derived from Coleoptera, an amino acid at position 205, or an amino acid corresponding to that at position 205 of luciferase from Genji firefly (*Luciola cruciata*) or Heike firefly (*Luciola lateralis*) is substituted by a different amino acid. Examples of such a different amino acid include an acidic amino acid such as aspartic acid or glutamic acid.

In another embodiment of the present invention, the above Coleoptera luciferase is a firefly luciferase such as a luciferase from Genji firefly (*Luciola cruciata*) or Heike firefly (*Luciola lateralis*).

In addition, in a second aspect, the present invention provides a variant luciferase gene encoding the amino acid sequence of any one of the above types of luciferase.

Moreover, in a third aspect, the present invention provides a recombinant DNA, which is characterized by insertion of the above variant luciferase gene into a vector DNA.

Furthermore, in a fourth aspect, the present invention provides a transformant or a transductant comprising the above recombinant DNA.

Still further, in a fifth aspect, the present invention provides a method for producing a variant luciferase, which comprises culturing the above transformant or transductant in a medium and collecting the variant luciferase from the culture product.

### DETAILED DESCRIPTION OF THE INVENTION

The present invention is described in detail below.

Since the variant luciferase of the present invention is provided by genetic recombination, a luciferase gene and its recombinant DNA (i.e. a vector DNA comprising a luciferase gene) need to be prepared. For such preparation, depending on the derivation or origin of luciferase genes, many types of corresponding variant genes can be designed and produced. Examples of luciferase genes include luciferase genes derived from Coleoptera, such as a luciferase gene derived from fireflies such as Heike firefly (*Luciola lateralis*), Genji firefly (*Luciola cruciata*) and North American firefly etc. (Tatsumi, H. *et al., Biochim Biophys Acta* 1131, 161-165 (1992); Masuda, T. *et al*., *Gene* 77, 265-270 (1989); de Wet, J. R. *et al., Mol. Cell Biol*. 7, 725-737 (1987)); a luciferase gene derived from *Pyrophorus noctilucus* (Wood, K. V. *et al., Science* 244, 700-702 (1989)); a luciferase gene derived from *Phrixpthrix* sp. (NCBI gene bank, accession number AF139644, AF139645) and so on. Otherwise, DNA encoding the variants of naturally occurring luciferase as above can also be used. Examples of such variants include: a variant luciferase of a wild type firefly luciferase, wherein an amino acid at position 233 in the amino acid sequence is substituted by isoleucine; one wherein an amino acid at position 239 in the amino acid sequence is substituted by isoleucine; one wherein an amino acid at position 286 in the amino acid sequence is substituted by asparagine; one wherein an amino acid at position 433 in the amino acid sequence is substituted by tyrosine; and one wherein an amino acid at position 452 in the amino acid sequence is substituted by serine (see Japanese Patent Application Laid-Open (kokai) No. 3-285683 (December 16, 1991)).

In a case where a gene of interest is known, a luciferase gene may be prepared according to a method described in the known document, or in a case where a gene of interest is unknown, it may be prepared, being amplified by a polymerase chain reaction (PCR), using primers prepared based on a common sequence among known luciferase genes. For example, the luciferase gene of Coleoptera can be prepared by a method described in Japanese Patent Application Laid-Open (kokai) No. 3-285683 (December 16, 1991), just as with a recombinant DNA thereof comprising the above gene. That is to say, mRNA is obtained from a solution containing the crushed dry tail of Coleoptera by performing n-butanol/chloroform treatment, precipitation with ethanol and oligo(dT)cellulose chromatography, and a protein encoded by the mRNA is assayed with anti-luciferase serum, thereby obtaining luciferase mRNA-rich fractions. Then, cDNA is prepared according to standard techniques, and the cloning of luciferase cDNA is performed (see, e.g. *Molecular Cloning* (1989), Second edition, Cold Spring Harbor Laboratory Press, USA).

In the present invention, the term "an amino acid corresponding to that at position 205 of luciferase from Genji or Heike firefly" is used to mean an amino acid corresponding to the amino acid at position 205 of the Genji or Heike firefly luciferase, when the amino acid sequence of Coleoptera luciferase is compared with the amino acid sequence of the Genji or Heike firefly luciferase. The amino acid sequence of the Genji or Heike firefly luciferase together with the corresponding nucleotide sequence is described in Tatsumi, H. *et al*., *Biochim Biophys Acta* 1131, 161-165 (1992) or Masuda, T. *et al*., *Gene* 77, 265-270 (1989). The 205^{th} position and the type of the amino acid in the present invention can be determined, for example, by analyzing the homology between the amino acid sequence of Coleoptera luciferase and that of the Genji or Heike firefly luciferase by ready-made software for analyzing the homology of amino acids (e.g. Micro Genie™ (Beckman)). Such a type of amino acid includes, but is not limited to, glycine.

Furthermore, "acidic amino acid" in the present invention includes aspartic acid, glutamic acid or the like. Among these, aspartic acid is particularly preferable. In the present invention, an amino acid can be substituted by amino acids other than an acidic amino acid, as long as sustention of the resulting luminescence can be improved when compared with that of the wild type luciferase.

Mutation treatment for the luciferase gene derived from Coleoptera or the like can generally be performed by a known method, depending on desired mutant forms. Such methods include a wide range of methods such as a method of allowing a luciferase gene derived from Coleoptera, or a recombinant DNA into which the above gene is incorporated, to be contacted with, and be acted upon a mutagenic agent; ultraviolet irradiation; genetic engineering techniques; or a method in which protein engineering techniques are applied.

Examples of agents serving as a mutagenic agent in the above mutagenic treatment include hydroxylamine, N-methyl-N'-nitro-N-nitrosoguanidine (NTG), nitrous acid, sulfurous acid, hydrazine, formic acid, 5-bromouracil and the like.

Conditions applied for the above contact and action depend on the type of agent to be applied, and are not particularly limited, as long as actually desired mutation can be provoked in a luciferase gene derived from Coleoptera or the like under those conditions. In general, the desired mutation can be provoked by contacting an agent to the gene to allow to act upon it at a reaction temperature of 20°C to 80° C for at least 10 minutes, preferably for 10 to 180 minutes at the preferable concentration of the above agent of 0.5M to 12M.

Also in a case where ultraviolet irradiation is applied, mutation treatment can be performed according to standard techniques as the above (see e.g. *Gendai Kagaku* (*Modern Chemistry*), page 24 to 30, June 1989, Tokyo Kagaku Dozin, Tokyo, Japan).

As a method in which a protein engineering technique is applied, a method generally known as Site-Specific Mutagenesis can be used. Examples of such methods include Kramer's method (Kramer, W. *et al., Nucleic Acids Res*, vol. 12, pp.9441-9456 (1984): Kramer, W. *et al., Methods Enzymol*, vol. 154, pp.350-367 (1987): Bauer, C. E. et *al., Gene*, vol. 37, pp.73-81 (1985)); Eckstein's method (Taylor, J. W. *et al., Nucleic Acids Res*, vol. 13, pp.8749-8764 (1985): Taylor, J. W. *et al., Nucleic Acids Res*, vol. 13, 8765-8785 (1985): Nakamaye, K. L. *et al.*, *Nucleic Acids Res*, vol. 14, pp.9679-9698 (1986)); and Kunkel's method (Kunkel. T. A., *Proc. Natl. Acid. Sci*. USA., vol. 82, pp.488-492 (1985): Kunkel. T. A. *et al., Methods Enzymol*, vol. 154, pp.367-382 (1987)) etc.

Moreover, the method generally known as Polymerase Chain Reaction can also be used (David, W. L. *et al., Technique*, vol. 1, No. 1, pp. 11-15 (1989)).

Furthermore, apart from the above-stated gene modification methods, the desirably modified Coleoptera luciferase gene may directly be synthesized by organic synthesis or enzyme-utilizing synthesis. In such a case, a commercially available nucleic acid synthesizer can be applied.

The determination and confirmation of the nucleotide sequence of the desired Coleoptera-derived luciferase gene obtained by the above methods can be performed, for example, by Maxam-Gilbert's chemical modification method (Maxam-Gilbert, *Meth. Enzym*., vol. 65, pp.499-560 (1980)), or by Dideoxynucleotide chain termination method wherein M13 phage is used (Messing *et al*., *Gene*, vol. 19., pp.269-276 (1982)).

According to standard techniques, the Coleoptera-derived luciferase gene obtained as the above can be incorporated into a vector such as a bacteriophage, cosmid or plasmid used for the transformation of a prokaryotic or eukaryotic cell, so that the host corresponding to each vector can be transformed or transducted (or transfected) according to standard techniques.

When a microorganism belonging to Escherichia such as *Escherichia coli* JM101 (ATCC 33876), *Escherichia coli* DH1 (ATCC 33849), *Escherichia coli* HB 101 (ATCC 33694) or the like is selected as a host, a transformed strain or a transduced strain can be obtained by transforming according to Hana-han's method (*DNA cloning* (1985) 1:109-135) and the like, or transducting according to a method described in *Molecular Cloning* (1989), Second edition, pp.256-268, Cold Spring Harbor Laboratory Press and the like.

Subsequently, the screening of the above strains results in obtainment of strains, which comprise a recombinant DNA obtained by the insertion of a variant luciferase gene into any appropriate vector DNA and have an ability to produce a variant luciferase.

Then, the novel purified recombinant DNA can be obtained from the above-obtained strains according to a method of P. Guerry *et al.* (*J. Bacteriology* (1973) 116:1064-1066), D. B. Clewell's method (*J. Bacteriology* (1972) 110:667-676) and so on.

To obtain DNA containing a variant luciferase gene from thus obtained recombinant DNA, for example, restriction enzymes such as *EcoR* I and *Pst* I are allowed to act on the above plasmid DNA at 30°C to 40°C, preferably at around 37°C, for 1 to 24 hours, preferably for about 2 hours, and then the reaction solution is subjected to agarose gel electrophoresis (*Molecular Cloning* (1982), p.150, Cold Spring Harbor Laboratory Press).

To produce a variant luciferase using a strain comprising a recombinant DNA comprising a vector DNA into which a variant luciferase gene is inserted, and having an ability to produce the variant luciferase, this strain can be cultured according to an ordinary solid or liquid culture method, preferably a liquid culture method.

An example of mediums used for the culture of the above strain includes a medium obtained by adding at least one inorganic salt such as sodium chloride, potassium dihydrogen phosphate, dipotassium monohydrogen phosphate, magnesium sulfate, magnesium chloride, ferric chloride, ferric sulfate or manganese sulfate, to at least one nitrogen source such as yeast extract, tryptone, peptone, meat extract, corn steep liquor, or the exudate of soybean or wheat bran. If necessary, sugar materials and vitamins are added to this medium.

The initial pH of the medium is preferably adjusted to pH 7 to 9. Moreover, the cultivation is performed at temperature of 20°C to 42°C, preferably at around 37°C, for 4 to 24 hours, preferably for 4 to 8 hours. Preferable culture methods include aeration-agitation submerged culture, shake culture, and static culture.

After completion of the culture, to collect a variant luciferase from the culture product, standard means for collecting enzymes can be employed.

That is, the present enzyme can be discharged out of the cells by disrupting the cells by ultrasonication or milling according to standard techniques; extracting the present enzyme with lytic enzymes such as lysozyme; or shaking or leaving the cells in the presence of toluene for autolysis. The treated solution containing the enzyme is filtrated or centrifuged to remove the solid matter, and if necessary, nucleic acids are also removed with streptomycin sulfate, protamine sulfate, manganese sulfate and the like. Then, fractionation is performed by adding ammonium sulfate, alcohol, acetone and the like. Thus, a crude enzyme can be obtained by collecting a precipitate.

To obtain an authentic enzyme sample, which is purifyed from the above crude enzyme, an appropriate method can be selected from such methods as: gel filtration using Sephadex, Ultrogel, Biogel and the like; absorption-elution using an ion exchanger; electrophoresis using polyacrylamide gel and the like; absorption-elution using hydroxyapatite; a sedimentation method such as sucrose density gradient centrifugation; affinity chromatography; and fractionation using a molecular-sieve membrane or a hollow fiber membrane, or several of the above methods can be used in combination.

Thus, the desired variant luciferase of the present invention can be obtained as above.

### EXAMPLES

The present invention is further described in the following examples. The examples are provided for illustrative purposes only, and are not intended to limit the scope of the invention.

### Example 1.

### Production of variant Genji firefly luciferase and titration

Genji firefly luciferase cDNA and Genji firefly luciferase expression plasmid pGLf37 were obtained by the method described in Examples of Japanese Patent Application Laid-Open (kokai) No. 3-285683 (December 16, 1991).

30 µg of the recombinant plasmid pGLf37 DNA was dissolved in 100 µl of a hydroxylamine solution (0.8M hydroxylamine hydrochloride, 0.1M phosphate buffer (pH6.0), 1mM EDTA), and the mixture was subjected to mutation treatment at 65°C for 2 hours, followed by precipitation with ethanol according to standard technique, so that precipitate was collected. This precipitate was dissolved in TE buffer (10mM Tris-hydrochloride buffer (pH7.5), 1mM EDTA), and was transformed into *Escherichia coli* JM 101 strain (ATCC 33876) by Hana-han's method (*DNA cloning* (1985) 1:109-135). The transformant was inoculated into an LB-amp agar medium (1% (w/v) bactotryptone, 0.5% (w/v) yeast extract, 0.5% (w/v) NaCl, 50 µg/ml ampicillin and 1.4% (w/v) agar) followed by culture at 37°C. After 12 hours, the colonies that appeared were subjected to shake culture at 37°C for 18 hours in 3ml of LB-amp medium (1% (w/v) bactotryptone, 0.5% (w/v) yeast extract, 0.5% (w/v) NaCl, and 50 µg/ml ampicillin). Then, 0.5ml of the cultured fluid was inoculated into 10ml of the above LB-amp medium and subjected to shake culture at 37°C for 4 hours, followed by centrifugation at 8,000r.p.m. for 10 minutes to obtain 20mg each of wet cells.

The collected cells were suspended into 0.9ml of buffer containing 0.1M KH₂PO₄ (pH7.8), 2mM EDTA, 1mM dithiothreitol and 0.2mg/ml protamine sulfate, and then 100 µl of 10mg/ml lysozyme solution was further added thereto, followed by leaving in ice for 15 minutes. Subsequently, the suspension was frozen in a bath containing methanol and dry ice, then left at 25°C to thaw completely. After that, centrifugation at 12,000 r.p.m. for 5 minutes was carried out to obtain 1ml of crude enzyme as a supernatant.

10 µl of the thus obtained crude enzyme solution containing a variant luciferase was reacted by a method described in Japanese Patent Application Laid-Open (kokai) No. 1-141592 (June 2, 1989), and its luminescent pattern was measured for 60 seconds using a luminometer (Aloka, luminescence reader BLR-201). Specifically, the titer of luciferase was determined as follows.

8ml of 25mM glycylglycine (pH7.8), 0.5ml of a magnesium sulfate solution (25mM glycylglycine (pH7.8) to which magnesium sulfate was added to a concentration of 0.1M), and 0.8ml of a luciferin solution (25mM glycylglycine (pH7.8) to which luciferin was added to a concentration of 1mM) were mixed, thereby preparing a luciferin mixture. 400 µl of thus obtained luciferin mixuture and 10 µl of luciferase to be measured were mixed. Then, 80 µl of an ATP solution (25mM glycylglycine (pH7.8) to which ATP was added to a concentration of 10mM) was poured into the obtained mixture, and immediately after this action, the number of generated photons was measured for each of 20 and 60 seconds, using a luminometer.

As a result of the above measurement, it became clear that a crude enzyme solution acting to bring about longer luminescence sustention when compared with a wild type Genji firefly luciferase had been obtained.

Then, this crude enzyme solution was purified as follows by the method described in Japanese Patent Application Laid-Open (kokai) No. 1-141592 (June 2, 1989).

The crude enzyme solution was salted out with ammonium sulfate, and the precipitate generated at 30% to 60% saturation was centrifuged at 30,000 r.p.m. for 10 minutes. The precipitate was dissolved in 25mM mixed solution (a small amount of 25mM Tris (hydroxy) aminomethane - hydrocloride buffer, to which 1mM disodium ethylene-diamine-tetraacetate salt, and ammonium sulfate to be at 10% saturation were added (pH7.3)), and a solution was obtained. Then, gel filtration chromatography was performed by passing the obtained solution through an Ultrogel AcA34 column (LKB) equilibrated with the above 25mM mixed solution, thereby obtaining active fractions. The thus obtained active fractions were dialyzed against phosphate buffer (10mM disodium monohydrogenphosphate-sodium dihydrogenphosphate solution, to which 0.1M sodium chloride and 10% (v/v) ethylene glycol were added), and the obtained solution was absorbed to a hydroxyapatite HPLC column (Tosoh, TSKgel HA-1,000) equilibrated with 10mM phosphate buffer, and elution was performed with linear gradient from 10mM to 100mM phosphate buffers (pH7.5), thereby obtaining a purified variant luciferase.

The luminescent pattern of the variant luciferase purified by the above method was determined by the above titration. As a result, it was found that the purified variant luciferase acted to bring about longer luminescence sustention when compared with a wild type Genji firefly luciferase.

That is to say, the above-obtained variant firefly luciferase is a luciferase, wherein glycine at position 205 is substituted by aspartic acid in the amino acid sequence of the wild type Genji firefly luciferase (Masuda, T. *et al.*, *Gene* 77, 265-270 (1989)). When the present purified enzyme was reacted by the above method (pH8.5) and its luminescent level was determined, the level was determined as 15.4Kcounts/mg protein for 20 seconds, and as 32.6Kcounts/mg protein for 60 seconds. When the wild type Genji firefly luciferase was subjected to the same reaction as a control, the luminescent level was 14.4Kcounts/mg protein for 20 seconds, and 23.9Kcounts/mg protein for 60 seconds. As is clear from the difference between the two types of luciferase, the variant luciferase of the present invention brings about sustained luminescence over a longer period of time (about 1.4 times of luminescent level for 60 seconds) when compared with a wild type luciferase (control), i.e., the luminescent level of the former is at least about 1.4 times higher than that of the latter when the determination is performed for 60 senconds.

In the above examples, the effect of a variant Genji firefly luciferase was confirmed. However, similar effect can be expected for other variant luciferases of the present invention, which are prepared based on the sequences of luciferases from Coleoptera other than Genji firefly, e.g. Heike firefly, North American firefly and *Pyrophorus noctilucus*, and also the sequences of other variant firefly luciferase sequences (e.g. variant luciferase encoded by each DNA of pGLf37C-M-2 (accession No. FERM BP-2825), pGLf37C-M-5 (accession No. FERM BP-3136), pGLf37C-M-4 (accession No. FERM BP-2826), pGLf37C-M-1 (accession No. FERM BP-3135), pGLf37C-M-6 (accession No. FERM BP-3137), pGLf37C-M-7 (accession No. FERM BP-3138), described in Japanese Patent Application Laid-Open (kokai) No. 3-285683 (December 16, 1991)).

Recombinant plasmid DNA, into which a gene encoding a variant luciferase was incorporated, was named as pGLf37-205D, and *Escherichia coli* transformed by the recombinant plasmid DNA, i.e., *Escherichia coli* JM101 (pGLf37-205D) was deposited under the terms of the Budapest Treaty, with an international depositary authority, the National Institute of Bioscience and Human-Technology, Agency of Industrial Science and Technology (1-1-3 Higashi, Tsukuba-shi, Ibaragi-prefecture, Japan (ZIP 305-0046)) under accession No. FERM BP-4929.

### Industrial Applicability

According to the present invention, a variant luciferase (especially one which is derived from Coleoptera) acting to bring about stably sustained luminescence for a long period of time is provided. Using this prolonged reaction of luciferase, it becomes possible to perform a highly sensitive determination of ATP or the like, and also to impart sustained luminescence to luminous plaything. Furthermore, according to the present invention, it becomes also possible to efficiently produce a variant luciferase having improved luminescence sustention.

## Claims

1. A variant luciferase having improved luminescence sustention when compared to a wild type luciferase, wherein, in the amino acid sequence of luciferase derived from Coleoptera, an amino acid at position 205, or an amino acid corresponding to that at position 205 of luciferase from Genji firefly (Luciola cruciata) or Heike firefly (Luciola lateralis) is substituted by a different amino acid, wherein said different amino acid is an acidic amino acid.

2. The variant luciferase according to claim 1 wherein the acidic amino acid is aspartic acid or glutamic acid.

3. The variant luciferase according to claim I wherein the luciferase derived from Coleoptera is a firefly luciferase.

4. The variant luciferase according to claim 3 wherein the firefly luciferase is a luciferase derived from Genji firefly (Luciola cruciata) or Heike firefly (Luciola lateralis).

## Patentansprüche

1. Luciferasevariante mit verbesserter Lumineszenz-Aufrechterhaltung als Luciferase vom Wildtyp, worin in der Aminosäuresequenz von Luciferase, die von Coleoptera stammt, eine Aminosäure an Position 205 oder eine Aminosäure, die dieser entspricht, an Position 205 von Luciferase eines Genji-Leuchtkäfers (Luciola cruciata) oder Heike-Leuchtkäfers (Luciola lateralis) durch eine andere Aminosäure substituiert ist, worin die andere Aminosäure eine saure Aminosäure ist.

2. Luciferasevariante nach Anspruch 1, worin die saure Aminosäure Asparaginsäure oder Glutaminsäure ist.

3. Luciferasevariante nach Anspruch 1, worin die von Coleoptera stammende Luciferase eine Leuchtkäfer-Luciferase ist.

4. Luciferasevariante nach Anspruch 3, worin die Leuchtkäfer-Luciferase eine von einem Genji-Leuchtkäfer (Luciola cruciata) oder Heike-Leuchtkäfer (Luciola lateralis) stammende Luciferase ist.

## Revendications

1. Variant de luciférase ayant une meilleure conservation de luminescence en comparaison avec une luciférase du type sauvage où, dans la séquence d'acides aminés de la luciférase dérivée de Coleoptera, un acide aminé à la position 205 ou un acide aminé correspondant à celui à la position 205 de la luciférase de la luciole de Genji (Luciola cruciata) ou de la luciole de Heike (Luciola lateralis) est substitué par un acide aminé différent, où ledit acide aminé différent est un acide aminé acide.

2. Variant de luciférase selon la revendication 1 où l'acide aminé acide est l'acide aspartique ou l'acide glutamique.

3. Variant de luciférase selon la revendication 1 où la luciférase dérivée du Coleoptera est une luciférase de la luciole.

4. Variant de luciférase selon la revendication 3 où la luciférase de la luciole est une luciférase dérivée de la luciole Genji (Luciola cruciata) ou la luciole Heike (Luciola lateralis).
